# EUROPEAN PATENT SPECIFICATION

(11) **EP 2 138 844 B1**
(45) Date of publication and mention of the grant of the patent: **25.04.2012**
(21) Application number: 08011630.4
(22) Date of filing: 26.06.2008
(51) Int. Cl.: G01N 33/50

(54) **Method for testing the response of cells to exposures with therapeutics**
Verfahren zum Testen der Reaktion von Zellen auf die Einwirkung von Therapeutika
Procédé pour tester la réponse de cellules aux expositions des thérapies

(43) Date of publication of application: 30.12.2009
(73) Proprietor: SpheroTec GmbH, 82152 Martinsried (DE)
(72) Inventor: Mayer, Barbara, 87700 Memmingen (DE)
(74) Representative: Stolmár, Matthias

(56) References cited:
- M.JOKA, HEINEMANN, R.ISSELS, K.JAUCH, B.MAYER: "prediction of the therapeutic repsonse in colorectal cancer patients using spheroid microtumor technology" JOURNAL OF CLINICAL ONCOLOGY, vol. 26, no. 15S, 20 May 2008 (2008-05-20), page 22178, XP002509449 Retrieved from the Internet: URL:http://meeting.ascopubs.org/cgi/conten t/abstract/26/15_suppl/22178> [retrieved on 2009-01-08]
- MAYER K ET AL: "631 Tumor associated fibroblasts have a profound impact on drug sensitivity of gastric cancer cells" EUROPEAN JOURNAL OF CANCER. SUPPLEMENT, PERGAMON, OXFORD, GB, vol. 2, no. 8, 1 September 2004 (2004-09-01), page 191, XP004640075 ISSN: 1359-6349
- DATABASE BIOSIS [Online] BIOSCIENCES INFORMATION SERVICE, PHILADELPHIA, PA, US; October 2007 (2007-10), FRIEDRICH JUERGEN ET AL: "A reliable tool to determine cell viability in complex 3-D culture: The acid phosphatase assay" XP002509453 Database accession no. PREV200800060901 & JOURNAL OF BIOMOLECULAR SCREENING, vol. 12, no. 7, October 2007 (2007-10), pages 925-937, ISSN: 1087-0571
- DATABASE MEDLINE [Online] US NATIONAL LIBRARY OF MEDICINE (NLM), BETHESDA, MD, US; December 1982 (1982-12), JONES A C ET AL: "In vitro cytotoxic drug sensitivity testing of human tumour xenografts grown as multicellular tumour spheroids." XP002509456 Database accession no. NLM6891260 & BRITISH JOURNAL OF CANCER DEC 1982, vol. 46, no. 6, December 1982 (1982-12), pages 870-879, ISSN: 0007-0920
- WALKER T M ET AL: "The differential cytotoxicity of methotrexate in rat hepatocyte monolayer and spheroid cultures." TOXICOLOGY IN VITRO : AN INTERNATIONAL JOURNAL PUBLISHED IN ASSOCIATION WITH BIBRA OCT 2000, vol. 14, no. 5, October 2000 (2000-10), pages 475-485, XP002509478 ISSN: 0887-2333
- FENG XU ET AL: "Three-dimensional polymeric systems for cancer cell studies" CYTOTECHNOLOGY, KLUWER ACADEMIC PUBLISHERS, DO, vol. 54, no. 3, 31 July 2007 (2007-07-31), pages 135-143, XP019525901 ISSN: 1573-0778

## Description

The invention relates to methods for testing the response of spheroids to exposure with therapeutics. Preferably the spheroids are derived from primary isolate biological tissue and/or cell-containing bodily fluid. Further embodiments relate to spheroid sections and arrays including kits suitable for carrying out the method according to the present invention.

Patient oriented "tailored", "personalised", or "individualised" medicine is a new concept that has come to the horizon recently to identify suitable individualised treatment for each patient (see Jain KK, Curr Opin Mol Ther 4, 548 (2002)).

Within the state of the art, methods for determining the therapeutic efficiency of drugs or for the drug sensitivity are known. For example, clinical testing methods in which drugs are administered to patients and any change in an illness determined by diagnostic examination; *in vivo* (animal) testing methods in which drugs are administered to animals, often within whom exogenous (such as human) tissue is transplanted; cell culture testing methods in which cells are taken from a patient, brought into contact with a chemical or drug before or during cell culture.

Generally speaking the effect of a drug is known prior to administration to a patient. When a diagnosis is made, a patient undergoes a generalised standard treatment without considering individual variations in drug sensitivity. This is because current drug choice relies on results obtained through clinical trials using large populations of patients. Although various attempts have been made to predict individual responses, no universal methodology has been possible to date. This is due mainly to (1) the characteristics of cells, particularly cancer cells, which are not uniform between patients and may not even be uniform when isolated *in vitro* from surgically removed tissues or biopsy specimens, (2) the culture conditions do not generally mimic normal physiological surroundings, and (3) it has previously been extremely difficult to effectively and efficiently culture cells isolated from individual patients.

M. Joka et al. describe in J. Clinical Oncology 2008, vol. 26, No. 155 (May 20 Supplement) a method for the Prediction of the therapeutic response in colorectal cancer patients using the Spheroid Microtumor Technology. The Spheroid Microtumor Model was established using primary tumor tissue samples from CRC patients, where the spheroids were treated in vitro with drugs and molecular therapeutics.

Methods that attempt to individualise patient treatment or therapy are drug sensitivity testing in which a collagen gel substrate was used in place of a soft agar substrate in a primary culture system using human cancer cells (US 5,356,793), the exposure of thin 'slices' of cancer tissue to chemotherapeutic drugs *in vitro* (WO 2007/133551) or a ATP-TCA assay.

Personalised or individualised therapy is particularly important for cancer patients, not least because the chemotherapeutic anti-cancer drugs are known to induce severe side effects whilst varying in efficacy on a patient to patient basis. The harm to the patient from an aborted attempt at chemotherapy may be substantial: first, the patient has - 2a - been physically injured to some extent by the administration of the chemotherapy agents but has received little, if any, treatment benefit, secondly, in many cases, the cancer may progress during the period of ineffective treatment to a point where the therapy with the second-chosen group of agents comes too late to substantially prolong the patient's life. Not surprisingly, the methods already established within the state of art cannot overcome the problems associated with developing personalised treatment regimes. This is because these methods fail to replicate the biological responses and behaviours that naturally occur in the *in vivo* (bodily) environment. The characteristics of prior art methods are often dictated by the collective property of a cell population rather than by intrinsic features of individual cells.

As a result of these disadvantages there is a need for a reliable preclinical model both for drug testing and for the creation of individualised therapy that mimics the characteristics of the original somatic cells of the patient in their natural *in vivo* environment, for example in growth, metastasis and intrinsic resistance to various types of therapy drugs. Further, such a method should be easily and rapidly practicable and bear significantly lower costs than conventional (animal) experiments. Finally, there is a need for a method that can be performed with a high throughput thereby allowing testing for many different compounds at a time.

Spheroid models as three dimensional *in vitro* test systems have been found to allow rapid selection of promising therapeutic strategies. However, to date and for the reasons mentioned above, it has been extremely difficult to develop such spheroid models from primary isolates of biological tissue or cell containing bodily fluids. Surprisingly, it has now been found that by applying the method of the present invention it has been possible to prepare spheroids directly from primary isolate tissue without the need for intermediate cell culture steps or use of established cell culture lines. As a result the responses of a patient tissue to any kind of biological or chemical compound can be determined *in vitro* wherein the *in vitro* responses accurately reflect or mimic the *in vivo* responses. Moreover, by applying the method of the - 4 - present invention, the responses of patient tissue to multiple compounds can be tested concurrently to enable the development of personal or individualised treatment regimes.

### Summary of the Invention

In one aspect of the invention, cells directly derived from primary isolate biological tissue and/or cell-containing bodily fluid are cultured in a three dimensional environment under conditions that induce spheroid formation and the spheroids used to screen potential therapies by monitoring biological responses induced by exposure to therapeutics. The method comprises the steps of:
a) generating a single cell suspension from a biological tissue sample directly obtained without intermediate steps of subculture and removing apoptotic cells thereof;
b) generating at least one spheroid directly from the single cell suspension in a medium;
c) contacting the at least one spheroid with at least one therapeutic;
d) measuring the response of the at least one spheroid to the at least one therapeutic.

The invention further provides devices with fixed and mounted spheroids including spheroid arrays for carrying out the method of the present invention.

Finally the invention provides a method for diagnosing individuals who respond to a therapeutic strategy to select a suitable therapeutic and a method for designing individual therapy concepts.

### Detailed description of the invention

Throughout this application, various articles and patents are referenced. Disclosures of these applications in their entirety are hereby incorporated by reference into this application.

As used herein, the term "spheroid" refers to an aggregate, cluster or assembly of cells cultured to allow three-dimensional growth in contrast to the two-dimensional growth of cells in either a monolayer or cell suspension (cultured under conditions wherein the potential for cells to aggregate is limited). The aggregate may be highly organized with a well defined morphology or it may be a mass of cells that have clustered or adhered together with little organisation reflecting the tissue of origin. It may comprise a single cell type (homotypic) or more than one cell type (heterotypic). Preferably the cells are primary cells isolated from tissue but may also include a combination of primary isolates with an established cell line(s). Particular cell 'types' include, but are not limited to, somatic cells, stem cells, progenitor cells and cancer stem cells.

As used herein, the term "directly derived" refers to a suspension of single cells from a biological tissue and/or cell containing bodily fluid that has been obtained directly from an individual, donor or animal without intermediate steps of subculture through a series of cultures and/or hosts. Thus, a suspension of single cells is produced directly from the biological tissue and/or cell-containing bodily fluid. This is in contrast to established methods in which stable and highly passaged cell lines are used. Such cell lines are far removed from being directly derived from their progenitor tissue by several, often a great many, intermediate culture steps. By way of non-limiting example, sources of suitable tissues include, but are not limited to, benign or malignant primary and metastatic tissues, sources of suitable cell containing bodily fluids include, but are not limited to, pleural effusion fluid or ascites fluid (liquid tumors).

Preferably the cells originate from a mammal. Ideally the biological tissue sample is a primary isolate tissue sample.

As used herein, the term "primary isolate tissue sample" refers to biological tissue from a biological tissue and/or cell containing bodily fluid that has been obtained directly from, for example, an individual, patient or animal without intermediate steps of subculture through a series of cultures and/or hosts. Thus, in the method of the invention, a suspension of single cells is produced directly from the primary isolate tissue sample. This is in contrast to established cell lines used in the prior art and which are far removed from being directly derived from their progenitor tissue by several, often a great many, intermediate culture steps. By way of non-limiting example, sources of suitable tissues include, but are not limited to, benign/healthy or malignant primary tissues, metastatic tissues, sources of suitable cell containing bodily fluids include, but are not limited to, pleural effusion fluid or ascites fluid (liquid tumours).

The term "cell line" as used herein refers to cells derived from a primary culture by subculturing and that have exceeded the Hayflick limit. The Hayflick limit may be defined as the number of cell divisions that occur before a cell line becomes senescent or unable to replicate further. This limit is approximately 50 divisions for most non-immortalized cells and in terms of cell culture, equates to approximately 9 to 10 passages of cell subculture over the course of from about 12 to 14 weeks.

Primary tumours are tumours from the original site where they first developed. For example, a primary brain tumour is one that arose in the brain. This is in contrast to a metastatic tumour that arises elsewhere and metastasized (or spread) to, for example, the brain.

According to the present invention, the tissue which may be used for spheroid preparation may be a normal or healthy biological tissue, or may be a biological tissue afflicted with a disease or illness such as a tumour tissue or fluid derived from a tumour. Preferably the tissue is a mammalian tissue. Also encompassed are metastatic cells. The tissue may be obtained from a human, for example from a patient during a clinical surgery but also from biopsies. The tissue may also be obtained from animals such as mice, rats, rabbits, and the like. It is also possible according to the invention to prepare spheroids from stem cells, progenitor cells or cancer stem cells. In case the tissue is derived from clinical surgery, quantities of from 500 mg to 2 g are preferred, wherein quantities of from 750 mg to 1.5 g are particularly preferred. In case the tissue is derived from biopsies, quantities of from 50 mg to 500 mg are preferred, wherein quantities of from 75 mg to 300 mg are particularly preferred and quantities of from 100 mg to 250 mg are most preferred.

Besides cells originating from tumour tissue, other cells with various indications such as smooth muscle cells, adipocytes, neural cells, stem cells, islet cells, foam cells, fibroblasts, hepatocytes and bone marrow cells, cardiomyocytes and enterocytes are also encompassed within the present invention.

Also within the scope of the present invention is the possibility to rebuild a metastatic micro-tumour e.g. tumour cells with hepatocytes, or tumour cells with bone marrow cells.

Also useful within the invention are primary cancer cells such as gastric, colon and breast primary cancer cells and metastatic cells. Also encompassed by the invention are primary normal (healthy) cells such as endothelial cells, fibroblasts, liver cells, and bone marrow cells.

Preferably the cells are directly derived from the tissue of a patient or healthy donor, a tissue derived from surgery (e.g. surgical specimens), a biopsy, an aspiration or drainage and also cells from cell-containing body fluids.

The prior art methods disclose preparation of spheroids from well known cell lines which may be proliferated multiple times. As a result, the cell lines used in the prior art represent homogeneous cell lines which are not able to mimic a more heterogeneous *in vivo* cell system. Thus, the process of the present invention represents a significant step forward over the prior art since it has previously been extremely difficult to produce spheroids reliably and in useful quantities from "directly derived" or primary isolate samples.

Also within the scope of the invention are large spheroids which consist of a higher cell number in the range of from 10⁶ to 5x10⁶ cells. Large spheroids generally have a necrotic/apoptotic centre that correlates with the upregulation of various biomarkers such as HIF-1alpha, VEGF, TKTL-1 and others. Large and small spheroids are generally used for different purposes, for example, large spheroids may be used as a model of advanced tumors.

The present invention relates to methods of screening multicellular spheroids. In a first step of the method a suspension of single cells is prepared from at least one biological tissue and/or cell-containing bodily fluid in a medium. The concentration of cells in the suspension of single cells is adjusted in the range of from 10³ to 10⁷ cells/ml medium. 2 to 50 vol.-% of an inert matrix is then added to the suspension of single cells, which is then incubated, preferably in the presence of CO₂. The process is characterised in that the suspension of single cells is directly derived from at least one biological tissue and/or from at least one cell-containing bodily fluid.

In the process according to the invention the cells of the biological tissue and/or cell containing bodily fluid are first dissociated or separated from each other. Dissociation of the tissue is accomplished by any conventional means known to those skilled in the art. Preferably the tissue is treated mechanically or chemically, such as by treatment with enzymes. More preferably the tissue is treated both mechanically and enzymatically. Use of the term 'mechanically' means that the tissue is treated to disrupt the connections between associated cells, for example, using a scalpel or scissors or by using a machine, such as a homogenizer. Use of the term 'enzymatically' means that the tissue is treated using one or more enzymes to disrupt the connections between associated cells, for example, by using one or more enzymes such as collagenase, dispases, DNAse and/or hyaluronidase. Preferably a cocktail of enzymes is used under different reaction conditions, such as by incubation at 37°C in a water bath or at room temperature with shaking.

The dissociated tissue is then suspended in a medium to produce a suspension of single cells and from which the spheroids can be formed directly. It should be noted that prior art methods generally include a step of two-dimensional tissue culture in a growth medium prior to attempting three-dimensional cell cultivation. In two-dimensional culturing methods the cell culture adheres to the bottom of a vessel and is then removed for example using Trypsin, EDTA, Bionase or other suitable agents. After this 2D culture step, the suspension of single cells is prepared and from which the spheroids are produced. Thus, the two-dimensional culturing according to the prior art leads to a more or less homogeneous cell culture which is accordingly not able to mimic a heterogeneous *in vivo* cell system.

In contrast thereto, it has surprisingly been found that spheroids produced from suspensions of single cells prepared from primary isolate tissue according to the present invention retain essentially all of the biological properties of the originating biological tissue. The same applies when cell-containing bodily fluids are used.

Preferably the suspension of single cells is treated to remove dead and/or dying cells and/or cell debris. The removal of such dead and/or dying cells is accomplished by any conventional means known to those skilled in the art for example, using beads and/or antibody methods. It is known, for example, that phosphatidylserine is redistributed from the inner to the outer plasma membrane leaflet in apoptotic or dead cells. Annexin V and any of its conjugates which have a high affinity for phosphatidylserine can therefore be bound to these apoptotic or dead cells. The use of Annexin V-Biotin binding followed by binding of the biotin to streptavidin magnetic beads enables separation of apoptotic cells from living cells. Other suitable methods will be apparent to the skilled artisan. Surprisingly it has been found that as a result of the inclusion of this step, substantially all of the cells within the suspension of single cells are available to form spheroids with a biological profile or composition more closely mimicking that found *in vivo*.

Methods of the prior art often utilise a dye exclusion test to monitor the vitality or viability of cells. The dye exclusion test is used to determine the number of viable cells present in a cell suspension. It is based on the principle that live cells possess intact cell membranes that exclude certain dyes, such as trypan blue, eosin, or propidium iodide, whereas dead cells do not. In the trypan blue test, a cell suspension is simply mixed with dye and then visually examined to determine whether cells take up or exclude dye. A viable cell will have a clear cytoplasm whereas a nonviable cell will have a blue cytoplasm. Dye exclusion is a simple and rapid technique measuring cell viability but it is subject to the problem that viability is being determined indirectly from cell membrane integrity. Thus, it is possible that a cell's viability may have been compromised (as measured by capacity to grow or function) even though its membrane integrity is (at least transiently) maintained. Conversely, cell membrane integrity may be abnormal yet the cell may be able to repair itself and become fully viable. Another potential problem is that because dye uptake is assessed subjectively, small amounts of dye uptake indicative of cell injury may go unnoticed. In this regard, dye exclusion performed with a fluorescent dye using a fluorescence microscope may result in the scoring of more nonviable cells with dye uptake than tests performed with trypan blue using a transmission microscope. As a result of the use of this method, the suspensions of single cells and spheroids of the prior art comprise a far greater proportion of apoptotic or dead cells. This inclusion of dead matter means that the prior art spheroids are less able to mimic the conditions found in biological tissue *in vivo* which will lead to greater variability in, for example, the response of prior art spheroids to exposure with a therapeutic. This variability in turn leads to tighter constraints being imposed on the evaluation of any response of prior art spheroids to a therapeutic meaning that higher precision measurements are required. In contrast, because the spheroids of the present invention mimic the conditions found in biological tissue *in vivo* more accurately, any evaluation is not subject to such variability and tight constraints. Thus, when using the spheroids and method of the present invention it is possible to produce data which is as, or often more, accurate and/or meaningful than that of the prior art even when such evaluations are themselves' performed with less stringency, accuracy or precision.

A more sophisticated method of measuring cell viability is to determine the cell's light scatter characteristics, 7AAD or propidium iodide uptake. It will be apparent to one skilled in the art that use of a flow cytometer coupled with cell sorting may also accomplish removal of dead and/or apoptotic cells.

The suspension of single cells is prepared in a culture medium. The medium is designed such that it is able to provide those components that are necessary for the survival of the cells. Preferably the suspension of single cells is prepared in a medium comprising one or more of the following components: serum, buffer, interleukins, chemokines, growth factors, hydrogen carbonate, glucose, physiological salts, amino acids and hormones.

A preferred medium is RPMI 1640. RPMI 1640 was developed by Moore et. al. at Roswell Park Memorial Institute (hence the acronym RPMI). The formulation is based on the RPMI-1630 series of media utilizing a bicarbonate buffering system and alterations in the amounts of amino acids and vitamins. RPMI 1640 medium has been used for the culture of human normal and neoplastic leukocytes. RPMI 1640, when properly supplemented, has demonstrated wide applicability for supporting growth of many types of cultured cells.

Preferably, the medium further comprises L-glutamine, in particular a stabilized L-glutamine. L-glutamine is an essential nutrient in cell cultures for energy production as well as protein and nucleic acid synthesis. However, L-glutamine in cell culture media may spontaneously degrade forming ammonia as a by-product. Ammonia is toxic to cells and can affect protein glycosylation and cell viability, lowering protein production and changing glycosylation patterns. It is thus preferred that the L-glutamine is a stabilized glutamine, most preferably it is the dipeptide L-alanyl-L-glutamine, which prevents degradation and ammonia build-up even during long-term cultures. The dipeptide is commercially available as GlutamaxI®.

The medium may further comprise additional components such as antibiotics, for example, penicillin, streptomycin, neomycin, ampicillin, metronidazole, ciprofloxacin, gentamicin, amphotericin B, kanamycin, nystatin; amino acids such as methionine or thymidine; FCS and the like.

In addition to, or instead of, RPMI1640 other liquid media can be used, for example DMEM high or low glucose, Ham's F-10, McCOY's 5A, F-15, RPMI high or low glucose, Medium 199 with Earle's Salts or the different variants of MEM Medium.

Next, the concentration of cells in the suspension of single cells is adjusted to an appropriate cell concentration. An appropriate cell concentration means an amount of cells per milliliter of culture medium which supports the formation of spheroids in the incubation step. Appropriate cell amounts are preferably 10³ to 10⁷ cells/ml medium, more preferably 10³ to 5x10⁶ cells/ml medium and most preferred 10⁵ to 10⁶ cells/ml medium. Methods of determining cell concentration are known in the art, for example, the cells may be counted with a Neubauer counter chamber (hemocytometer).

Further, an appropriate amount of an inert matrix is added to the suspension of single cells. Use of the term "inert" as used herein refers to a matrix that has only limited or no ability to react chemically and/or biologically, i.e. having little or no effect on the biological behaviour/activity of the cells of the suspension of single cells. Preferably the inert matrix is of non-human origin.

Preferably the inert matrix increases the viscosity of the culture medium. Not wishing to be bound by theory, it is believed that increasing the viscosity of the culture fluid increases the co-incidental collision and adherence of cells with each other resulting in the formation of aggregates, possibly by reducing fluid shear. This is particularly useful since it improves the ability of shear sensitive or weakly adherent cells to aggregate and develop into spheroids.

Thus, the inert matrix 'supports' or aids the formation of spheroids during the incubation step. Preferably the inert matrix is added to the culture medium in an amount of 2 to 50 vol.-% based on the total volume of the medium. Preferably the inert matrix is added in an amount of 5 to 30 vol.-%, most preferably in an amount of 10 to 15 vol.-%. Particular amounts will vary depending on the source or composition of the cells such as 3, 4 or 5 vol.-% up to 10 or 15 vol.-% for cell lines and up to 30 to 45 or 50 vol.-% when using primary isolate tissue. These amounts are based on the total volume of the medium. The inert matrix is preferably a non-ionic poly(ethylene oxide) polymer, water soluble resin or water soluble polymer such as a cellulose ether. Preferably the inert matrix is selected from the group comprising carboxymethyl cellulose, hydroxypropyl cellulose, hydroxypropylmethyl cellulose, hypomellose, methyl cellulose, methylethyl cellulose. However, also suitable is cellulose, agarose, seaplaque agarose, starch, tragacanth, guar gum, xanthan gum, polyethylene glycol, and the like.

The single cell suspension is then incubated, preferably in the presence of CO₂. Incubation can also be carried out in the presence of water vapor. Possible preparation techniques are e.g. the liquid-overlay technique, the spinner flask technique, the high aspect rotating vessel (HARV) technique or the hanging drop method. These methods are known to the skilled artisan. The HARV technique is inter alia disclosed in US patents 5 153 131, 5 153 132, 5 153 133, 5 155 034, and 5 155 035. The spinner flask technique is disclosed in e.g. W. Mueller-Klieser, "Multicellular Spheroids", J. Cancer Res. Clin. Oncol., 12: 101-122, 1986. The liquid-overlay technique is disclosed e.g. in J.M. Yuhas et.al., "A simplified method for production and growth of multicellular tumor spheroids", Cancer. Res. 37: 3639-3643, 1977. The hanging drop method is disclosed in e.g. Bulletin of Experimental Biology and Medicine, Vol. 91, 3, 1981, Springer, New York. Most preferred in the present invention is the liquid-overlay technique. Generally these preparation techniques are all performed under CO₂ conditions.

The incubation may be performed at 30 °C to 45 °C, preferably at 37 °C, in a normoxic atmosphere containing about 4 to 6 vol.-% CO₂, preferably 5 vol.-% CO₂ or under hypoxic conditions, i.e. N₂ 92-95%, O₂ 5-8%. The incubation is performed of from about 5 hours to 9 days, preferably of from about 12 hours to 6 days, most preferred of from about 24 to 96 hours. However, it will be apparent to the skilled artisan that such temperatures and conditions will depend on the source and type of cells used and the treatment strategy.

As used herein, the term 'homotypic' refers to cells of a single type. For example, commercially available cell lines are generally homotypic. In contrast and as used herein, the term 'heterotypic' refers to cells of more than one cell type. For example, primary isolate tissue comprising different cell types will be heterotypic.

Thus, a homotypic spheroid could be prepared from one particular cancer cell line such as Hs746T, malignant primary cells isolated from primary or metastatic malignant tissues or healthy, benign primary cells such as fibroblasts, bone marrow cells, hepatocytes, other benign epithelial cells or chondrocytes. Heterotypic spheroids could be prepared from primary and metastatic patient tissue or from a combination of two or more homotypic cell lines such as a cancer cell line and benign primary cells.

Therefore, in another aspect of the present invention, a suspension of single cells may be combined with, for example, epithelial cells, immune cells, fibroblasts, hepatocytes, chondrocytes, bone marrow cells, airway epithelial cell cultures, enterocytes, cardiomyocytes, melanocytes, keratinocytes, adipocytes, stem cells, cancer stem cells and/or smooth muscle cells. It will be apparent to one skilled in the art that a suspension of single heterotypic cells may also be combined in this manner.

The advantage of combining homotypic cell types with epithelial cells, immune cells or fibroblasts (as well as other cell types cited above), is that tumour cells interact with epithelial cells, immune cells or fibroblasts (and also with such cells cited above) in nature. Hence, the combination with such cells leads to a heterotypic multicellular spheroid system which mimics even more closely an *in vivo* cell or metastic cell system.

The internal environment of a spheroid is dictated by the metabolism and adaptive responses of cells with a well-defined morphological and physiological geometry. Most homotypic spheroids develop concentric layers of heterogeneous cell populations with cells at the periphery and layers of quiescent cells close to a necrotic core. The heterogeneous arrangement of cells in a spheroid mimics initial avascular stages of early tumours. Although homotypic spheroids are able to closely mimic the in vivo morphology, some of the biological complexity is lost. Thus, by co-culture of more than one cell type in a spheroid, tumour cell interactions with other cell types reflecting natural cell interaction *in vivo* can be established better representing the *in vivo* environment.

Thus, suspensions of single cells may be combined with other cells, for example from established cell lines, primary cells generated from benign and/or malignant tissues. Most preferably the tissue is a tumour tissue wherein the cancer cell lines may be cell lines from gastric (e.g. Hs-746T, MKN-28, N87, and the like), colorectal (e.g. HT-29, HCT-116, DLD-1, and the like), liver (e.g. HepG2 and the like), pancreas (e.g. L.6pl, AsPC-1, miaPACA, and the like), lung (e.g. A549, H358, H1299, and the like), kidney (e.g. 786-O, A-498, CAKI-1, and the like), breast (e.g. MCF-7, BT549, Hs575T, and the like), cervical (e.g. HeLa and the like), prostate (e.g. PC-3, LNCaP, DU-145, and the like) or glioma (e.g. U251, U373, and the like) cell lines. It will be appreciated that the method is suitable for use with any cell line. In particular preferred are also cell lines from sarcoma or astrocytoma tissue.

Multicellular spheroids may comprise a single cell type (homotypic) or a mixture of two or more cell types (heterotypic).

The process as set forth above leads to spheroids with a nearly homogenous spherical shape, wherein the average diameter of the spheroids reaches from 50 to 2000 µm, preferably from 150 to 1000 µm and most preferred from 200 to 500 µm.

The multicellular spheroids according to the invention can also be characterized in that they exhibit characteristics that substantially mimic those of the tissue of origin such as in terms of one or more of the following: antigen profile and/or genetic profile, tumour biologic characteristics, tumour architecture, cell proliferation rate(s), tumour microenvironments, therapeutic resistance and composition of cell types, etc. Preferably, they exhibit an antigen profile and genetic profile which is substantially identical to that of the tissue of origin.

Thus, the spheroids of the invention exhibit a substantially similar/identical behaviour to that of natural cell systems, e.g. with respect to organization, growth, viability, cell survival, cell death, metabolic and mitochondrial status, oxidative stress and radiation response as well as drug response.

Since the multicellular spheroids according to the invention are substantially identical to *in vivo* cell systems, these spheroids can thus be used for diagnostic and/or therapeutic purposes, pharmacokinetic profiling, pharmacodynamic profiling, efficacy studies, cytotoxicity studies, penetration studies of compounds, therapeutic resistance studies, antibody testing, personalised or tailored therapies, RNA/DNA 'drug' testing, small molecule identification and/or testing, biomarker identification, tumour profiling, hyperthermia studies, radioresistance studies and the like.

In one aspect, the multicellular spheroids can be obtained from benign or malignant tissues or from primary cells and used for the screening of compounds, for example, as new therapeutic agents or screening for e.g. chemotherapeutics wherein the response of the spheroid to the chemotherapeutic can be determined. It is thus possible to see whether a chemotherapeutic has an effect and/or side effects on the multicellular spheroid, e.g. whether it causes cell death (apoptosis) or other biologic effect.

In a next step of the method of the present invention, the multicellular spheroids are contacted with at least one therapeutic. In the sense of the present invention, the term "therapeutic(s)" includes all biological or chemical agents and means of therapy used to treat disease. It is thereby understood that the term "at least" encompasses the use of one, more or combinations of different therapeutics as defined below.

It is thereby preferred that the at least one therapeutic is selected from the group of chemotherapeutic, cytostatics, biomarker and molecular therapeutics, but also includes the means of radiotherapy and hyperthermy.

In its non-oncological use, the term "therapeutic(s)" may also refer to antibiotics (antibacterial chemotherapy). Other uses of cytostatic chemotherapy agents are the treatment of autoimmune diseases such as multiple sclerosis and rheumatoid arthritis, viral infections, heart diseases and the suppression of transplant rejections. It will of course be apparent to the skilled artisan that such chemotherapeutics need not be limited to substances used to treat disease. Thus, the term may be applied more loosely to refer to any agent that the skilled person wishes to expose the spheroids to determine whether said agent has an effect, for example, on the behaviour or biological characteristics of the spheroids.

By way of non-limiting example, therapeutics may include, but are not limited to: alkylating agents, antimetabolites, anthracyclines, plant alkaloids, topoisomerase inhibitors, and other anti-tumour agents, antibodies such as monoclonal, single chain or fragments thereof and the new tyrosine kinase inhibitors e.g. imatinib mesylate (Gleevec® or Glivec®), small molecules, nucleic acids, tyrosine kinase receptor inhibitors, anticalins, aptamers, peptides, scaffolds, biosimilars, generic drugs, siRNA and RNA or DNA based agents.

"Means of therapy" used to treat disease include indirect exposure to an agent, for example using electromagnetic, non-ionising radiation (for example heat, microwaves, visible light, etc.), ionising radiation (e.g. UV, X-rays, Gamma radiation, etc.) or particle radiation (e.g. alpha, beta, etc.). The irradiation such as from radionuclides may be external or internal.

The use of such treatment may be especially beneficial as some therapeutics can act as radiosensitisers, for example, as described in WO 00/01411, enhancing the therapeutic effect of such irradiation. Other therapeutics may be prodrugs that do not become active until exposed to, for example, light or heat.

In yet other embodiments, the method may also involve indirect exposure, such as irradiation, of the at least one spheroid.

The number of spheroids used for testing a single compound may be from 1 to 10¹⁰, more preferably from 10² to 10⁵ and still more preferably from 10² to 10³.

Within the method of the present invention, the contact of the at least one spheroid with the at least one therapeutic may last between about 1 second and 14 days, more preferably between about 1 hour and 84 hours, still more preferably between about 12 hours and 60 hours and most preferably about 24 hours to 48 hours.

Within the method of the present invention, the temperature during contacting of the at least one spheroid with the at least one therapeutic is preferably between 10 °C and 70 °C, more preferably between 20 °C and 50 °C and most preferably between 35 °C and 39 °C wherein 37 °C is particularly preferred.

The concentration of the at least one therapeutic used within the method of the present invention is preferably chosen according to the concentration applied to a patient when medically treated. However, much lower or higher doses can also be applied when testing toxicity and/or effective levels of a drug. The doses are preferably chosen within a range of from 0.1 µmol to 15 µmol, more preferred of from 0.5 µmol to 10 µmol and most preferred of from 0.5 µmol to 7 µmol, however, depending on the particular therapeutic(s) used either in mono-therapy or in combination therapy.

It will be apparent to one skilled in the art that the parameters characterised above are primarily for guidance. Thus, the parameters may be used in any particular combination and will depend, for example, on the cell types and therapeutic(s) being used.

In a last step of the method, the response(s) of the at least one spheroid to the at least one therapeutic is/are determined to see whether the therapeutic has had any effect on the multicelluar spheroid, for example whether it causes cell death (apoptosis) or other biologic effects.

The response of the at least one spheroid is preferably determined through the use of biomarkers.

Biomarkers are generally characteristics that are objectively measured and evaluated as indicators of normal biologic processes, pathogenic processes or pharmacologic responses to a therapeutic intervention. Within the present invention, generally any biomarker(s) known to a person skilled in the art as suitable for the inventive purpose can be used, wherein protein, molecular or genomic biomarker(s) are preferred. Within each group prognostic, therapeutic or predictive biomarker(s) are particularly preferred. Most preferred, the biomarker(s) used within the present invention are selected from CEA, CA19.9, CA242, TIMP-1, TS, MSI, DCC, Ras, P53, FOBT, DNA-based markers, K-ras, APC, p53, L-DNA, BAT 26, LDH, GCC, MSI, LOH 18q, thymidylate synthase TS, thymidine phosphorylase TP, circulating tumor cells, gene expression profiling, CA 19-9, ERCC1 expression, skin rash, EGFR copy number, EGFR ligand expression, hENT-1 expression, RRM1 expression, MGMT expression, dihydropyrimidine dehydrogenase DPD, XPD, BRCA1/2, UGT1A1 polymorphism, UGT1A1 mutation, Ki67, B-RAF exon 11, B-RAF exon 15, micro satellite stability, FISH for Her2Neu, EpCAM, CD44v6, MUC-1, uPA, uPAR, EGF-R, insulin-like growth factor-1, VEGF-A, VEGF-R, HGF-R, Raf-B, p70S6-kinase, Hsp90, TRAIL-1. Further, spheroids as described within the present invention can be used as biomarker(s), particularly as functional biomarker(s).

By way of example, indirect measurement could include the use of rate of substrate depletion to monitor enzyme levels whereas direct measurement would involve measurement of enzyme levels per se. Biomarkers may be detectable and/or measurable by a variety of methods including laboratory assays such as nucleic acid analysis, HPLC and ELISA, analytical assays such as LC-MS, MALDI-TOF, LT-Q-FTMS, cytometry and microarrays or imaging methods including optical, ultrasound, X-ray, CT, PET, SPECT, immunohisto chemistry, PCR, mutation analysis, sequencing and MRI.

Biomarkers validated by genetic and molecular biology methods may be classified into a number of types such as:

| | |
|---|---|
| Type 0: | natural history markers; |
| Type 1: | drug activity markers; |
| Type 2: | surrogate markers. |

Suitable biomarkers of cancer may include, but are not limited to, cancer antigen 125 (CA-125) (as a biomarker of ovarian - 24 - cancer risk or an indicator of malignancy), carcinoembryonic antigen (CEA) (elevated in patients with colorectal, breast, lung, or pancreatic cancer), Prostate Specific Antigen (PSA) (prostate cancer) or Tyrosinase (melanoma).

The present invention further provides a method for diagnosing individuals who respond to a chemical or biological therapeutic strategy comprising the steps of:
a) generating a single cell suspension from a biological tissue sample directly obtained without intermediate steps of subculture and removing apoptotic cells thereof;
b) generating at least one spheroid directly from the single cell suspension in a medium;
c) contacting the at least one spheroid with at least one therapeutic;
d) measuring the response of the at least one spheroid to the at least one therapeutic;
wherein the response of the at least one spheroid to the at least one therapeutic is used to select a therapeutic agent.

Further to the method as described above, a post-therapeutic screening can be conducted by obtaining a second tissue sample from the same individual. The second tissue sample can thereby be taken either from the same tissue as the first sample or e.g. from metastatic tissue or secondary tumours. Thus, information about the success of the therapy can be obtained.

It should be understood that the method of the invention is not limited to screening/testing diseased cells. For example a combination of several tumour markers may be used to evaluate risk of developing a disease such as in someone whose family history for the disease is quite high.

Within the method of the present invention, the contact of the at least one spheroid with the at least one therapeutic can be divided in two or more sub-steps if one or more than one therapeutic is tested. It is thus possible to first evaluate the tissue with respect to at least one biomarker to detect the type of cell or tissue and/or the predictivity of the therapeutic response of the individual patient tissue(s), respectively, and then, in a second, third or further steps, testing one or different or combinations of therapeutics. The selection of the at least one therapeutic or combination thereof may thus be specifically tailored after determining the exact cell type and characteristics of the patient tissues.

The present invention therefore further provides a method for designing an individual therapy concept for a patient, comprising the steps of:
a) executing a biomarker profile for a tissue sample;
b) generating a single cell suspension from a biological tissue sample directly obtained without intermediate steps of subculture and removing apoptotic cells thereof;
c) generating at least one spheroid directly from the single cell suspension in a medium;
d) contacting the at least one spheroid with at least one therapeutic;
e) measuring the response of the at least one spheroid to the at least one therapeutic.

The term "biomarker" is thereby defined as above and preferably selected from the group of protein biomarkers, molecular biomarkers and genomic biomarkers.

The biomarker profile makes it thus possible to preselect the therapeutic(s) according to the characteristics of the individual tissue and cells.

Within a further step of this method of the present invention, a biomarker profile of the spheroid is carried out. The biomarker profile of the spheroid can thereby be carried out before and/or after the contact with the therapeutic(s), but is preferably carried out before and after the contact of the spheroid with the therapeutic(s). It is thereby possible to assess, whether the therapeutic(s) modify the characteristics of the original tissue and/or cells.

Further to the method as described above, a post-therapeutic screening can be conducted as described in the foregoing.

In another aspect of the present invention, the biomarker may be a measure of apoptosis. There are many ways of detecting apoptosis at different stages such as on histological sections and it is possible to apply any method known to the man skilled in the art. It is, however, preferred to use the "TUNEL" (Terminal deoxynucleotidyl Transferase Biotin-dUTP Nick End Labelling) method.

One of the characteristics of apoptosis is the degradation of DNA after the activation of Ca/Mg dependent endonucleases. This DNA cleavage leads to strand breaks within the DNA. The TUNEL method identifies apoptotic cells in situ by using terminal deoxynucleotidyl transferase (TdT) to transfer biotin-dUTP to these strand breaks of cleaved DNA. The biotin-labelled cleavage sites are then detected by reaction with HRP conjugated streptavidin and visualized by DAB showing brown colour. Recently, more methods for the detection of apoptotic cells and specific parts of the apoptotic pathway are also available such as the detection of caspase activity (specifically caspase-3), fas-ligand and annexin V, etc. It is also important to use TUNEL in conjunction with other methods especially morphology, immunohistochemistry and electron microscopy.

The at least one spheroid may be firmly fixed and/or held within a device, such as preferably a paraffin or cryo ice block. A sliced specimen preparation, known as a section, can then be made from the block and fixed on a microscope slide.

Paraffin in the sense of the present invention is a name for a group of alkane hydrocarbons with the general formula CₙH₂ₙ₊₂ wherein n is the number of carbon atoms. Within the scope of the present invention the solid forms of paraffin are preferred. The solid forms, also called "paraffin wax" are from the heaviest molecules from C₂₀ to C₄₀. Alternatives to paraffin will be apparent to one skilled in the art.

It will also be apparent to the skilled person that analysis of such sections is limited since following such embedding and sectioning the cells of the spheroid are no longer living cells. Thus, in this example, preferably the spheroid has been contacted with the at least one therapeutic prior to embedding and sectioning. Using a slide of such sections it is possible to carry out *in situ* hybridisation analysis and other analyses such as immunological staining procedures. Such slides will contain at least one section but may contain several tens or even hundreds of such sections in an ordered "spheroid array".

In further embodiments of the invention, spheroids may be formed, fixed, held and/or localised within microfabricated structures. Such structures may include, but are not limited to, plasma etched glass or silicon slides and the like. For example, a device may be constructed with an array of pyramid-like microwells in a silicon chip. Each microwell may be further provided with one or more microchannels. Spheroid cultures can then be introduced to the chip and due to the size of each microwell, a single spheroid deposited or localized precisely within each microwell. Microchannels may be used to replace culture medium allowing culture on the chip. Such a microfluidic structure enables the rapid testing or screening of 'live' spheroids in an ordered fashion with a reduced space requirement.

It will be apparent to one skilled in the art that such ordered arrays of spheroids may also be formed in, for example, paraffin which would allow sectioning of multiple, ordered spheroids at one time following exposure to at least one therapeutic. Each microarray block may be cut into 100 - 500 sections, which can each be subjected to independent tests.

### Brief description of Figure

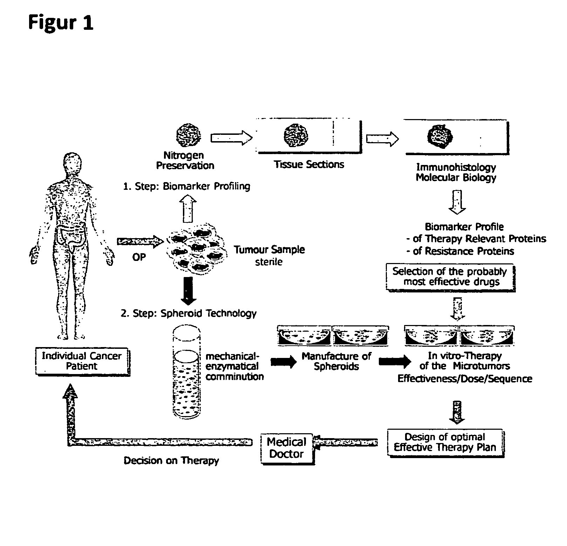

Figure 1 is a schematic diagram to illustrate optimisation of a therapeutic strategy for a patient using the method of the present invention.

### Example

The present invention will now be illustrated further by way of a non-limiting example:

### Preparation of heterotypic spheroids derived from primary patient tumour tissue.

A gastric cancer tissue with a size of about 0.5 cm³ was taken from a patient. The tissue was made into a suspension of single cells by reducing the tissue into small pieces with the aid of a scalpel and subsequent treatment with an enzyme cocktail consisting of collagenase, dispase, DNAse and hyaluronidase. Then, the cells were suspended in RPMI 1640 culture medium containing Glutamax I^{™} or L-Glutamine.

The viability of the cells was tested with the trypan-blue exclusion test and the cell number was adjusted to 10⁶ cells/ml medium with the aid of a Neubauer counter chamber. Cellulose ether was then added to the cell suspension and the suspension transferred to a 96-well plate with the following amounts of reagents:

For each well plate 12 ml cell suspensions were prepared (96 well x 100 µl/well = -10 ml + 2 ml excess = 12 ml), to provide a concentration of cells of 5 x 10⁴ cells/100 µl medium corresponding to 5 x 10⁵/ml corresponding to 6 x 10⁶/12ml.

The final suspension contained 6 ml of cell suspension, 5,4 ml RMPI 1640 + Glutamax^{™} and 0,6 ml cellulose ether (=5%)

The cell suspension was mixed and then transferred with a multichannel pipette to a 96-well plate in an amount of 100 µl/well. The cell suspension was then incubated at 37 °C in the presence of 5 % CO₂ for 24 hours.

After 24 hours, multicellular spheroids had formed and exhibited a homogeneous shape with a mean diameter of about 250 µm.

While certain of the preferred embodiments of the present invention have been described and specifically exemplified above, it is not intended that the invention be limited to such embodiments.

## Claims

1. Method of testing the response of cells to exposure with at least one therapeutic, comprising the steps of:
a) generating a single cell suspension from a biological tissue sample directly obtained without intermediate steps of subculture and removing apoptotic cells thereof;
b) generating at least one spheroid directly from the single cell suspension in a medium;
c) contacting the at least one spheroid with at least one therapeutic;
d) measuring the response of the at least one spheroid to the at least one therapeutic.

2. Method according to claim 1, wherein the tissue originates from a mammal.

3. Method according to claim 1 or 2, wherein the biological tissue sample is selected from healthy tissue, a tumour tissue, tissue from a benign or malignant tissue.

4. Method according to any of claims 1 to 3, wherein the at least one biological and/or chemical compound is selected from the group of chemotherapeutics, biomarkers and molecular therapeutics.

5. Method according to any of claims 1 to 4, wherein the at least one spheroid is also treated with radiation and/or hypothermia.

6. Method according to any of claims 1 to 5, wherein the contacting of the at least one spheroid with the at least one therapeutic lasts from 12 hours to 120 hours.

7. Device for carrying out a method according to any of claims 1 to 6, wherein at least one spheroid according to any of claims 1 to 6 is firmly fixed and/or held within a device.

8. Device according to claim 7, wherein the spheroid is a spheroid treated with at least one therapeutic.

9. Device according to claim 7 or 8, **characterised in that** the at least one spheroid is fixed and/or held by embedding in a paraffin or cryo ice block.

10. Method for diagnosing individuals who respond to a chemical or biological therapeutic strategy comprising the steps of:
a) generating a single cell suspension from a biological tissue sample directly obtained without intermediate steps of subculture and removing apoptotic cells thereof;
b) generating at least one spheroid directly from the single cell suspension in a medium;
c) contacting the at least one spheroid with at least one therapeutic;
d) measuring the response of the at least one spheroid to the at least one therapeutic;
wherein the response of the at least one spheroid to the at least one therapeutic is used to select a therapeutic agent.

11. Method for designing an individual therapy concept for a patient, comprising the steps of:
a) executing a biomarker profile for the tissue sample;
b) generating a single cell suspension from a biological tissue sample directly obtained without intermediate steps of subculture and removing apoptotic cells thereof;
c) generating at least one spheroid directly from the single cell suspension in a medium;
d) contacting the at least one spheroid with at least one therapeutic;
e) measuring the response of the at least one spheroid to the at least one therapeutic.

12. Method according to claim 11, wherein the biomarker(s) is/are selected from the group of protein biomarkers, molecular biomarkers and genomic biomarkers.

13. Method according to claim 11 or 12, wherein the therapeutic is selected according to the executed biomarker profile.

14. Method according to any of claims 11 to 13, further comprising the step of executing a biomarker profile of the spheroid.

## Patentansprüche

1. Verfahren zum Testen des Ansprechens von Zellen auf eine Exposition mit mindestens einem Therapeutikum, umfassend die Schritte:
a) des Erzeugens einer Einzelzellaufschwemmung aus einer biologischen Gewebeprobe, die direkt ohne Subkulturzwischenschritte gewonnen wurde, und des Entfernens von apoptotischen Zellen aus derselben;
b) des Erzeugens mindestens eines Sphäroids direkt aus der Einzelzellaufschwemmung in einem Medium;
c) des In-Kontakt-Bringens zwischen dem mindestens einen Sphäroid und dem mindestens einen Therapeutikum;
d) des Messens des Ansprechens des mindestens einen Sphäroids auf das mindestens eine Therapeutikum.

2. Verfahren nach Anspruch 1, wobei das Gewebe aus einem Säugetier stammt.

3. Verfahren nach Anspruch 1 oder 2, wobei die biologische Gewebeprobe aus einem gesunden Gewebe, einem Tumorgewebe, Gewebe aus einem gutartigen oder malignen Gewebe ausgewählt ist.

4. Verfahren nach einem der Ansprüche 1 bis 3, wobei die mindestens eine biologische und/oder chemische Verbindung aus der Gruppe der Chemotherapeutika, Biomarker und molekularen Therapeutika ausgewählt ist.

5. Verfahren nach einem der Ansprüche 1 bis 4, wobei das mindestens eine Sphäroid auch mittels Bestrahlung und/oder Hypothermie behandelt wird.

6. Verfahren nach einem der Ansprüche 1 bis 5, wobei das In-Kontakt-Bringen zwischen dem mindestens einen Sphäroid und dem mindestens einen Therapeutikum über eine Dauer von 12 Stunden bis 120 Stunden erfolgt.

7. Vorrichtung zum Durchführen einer Verfahren nach einem der Ansprüche 1 bis 6, wobei mindestens ein Sphäroid nach einem der Ansprüche 1 bis 6 fest in einer Vorrichtung fixiert ist und/oder in einer Vorrichtung gehalten wird.

8. Vorrichtung nach Anspruch 7, wobei das Sphäroid ein Sphäroid ist, das mit mindestens einem Therapeutikum behandelt wurde.

9. Vorrichtung nach Anspruch 7 oder 8 **dadurch gekennzeichnet, dass** das mindestens eine Sphäroid mittels Einbettung in einen Paraffin- oder Kryoeisblock fixiert ist und/oder gehalten wird.

10. Verfahren zur Diagnostik von Personen, die auf eine chemische oder biologische therapeutische Strategie ansprechen, umfassend die Schritte:
a) des Erzeugens einer Einzelzellaufschwemmung aus einer biologischen Gewebeprobe, die direkt ohne Subkulturzwischenschritte gewonnen wurde, und des Entfernens von apoptotischen Zellen aus derselben;
b) des Erzeugens mindestens eines Sphäroids direkt aus der Einzelzellaufschwemmung in einem Medium;
c) des In-Kontakt-Bringens zwischen dem mindestens einen Sphäroid und dem mindestens einen Therapeutikum;
d) des Messens des Ansprechens des mindestens einen Sphäroids auf das mindestens eine Therapeutikum;
wobei das Ansprechen von mindestens einem Sphäroid auf das mindestens eine Therapeutikum verwendet wird, um ein therapeutisches Mittel auszuwählen.

11. Verfahren zum Entwerfen eines individuellen Therapiekonzepts für einen Patienten, umfassend die Schritte:
a) des Erstellens eines Biomarkerprofils für die Gewebsprobe;
b) des Erzeugens einer Einzelzellaufschwemmung aus einer biologischen Gewebeprobe, die direkt ohne Subkulturzwischenschritte gewonnen wurde, und des Entfernens von apoptotischen Zellen aus derselben;
c) des Erzeugens mindestens eines Sphäroids direkt aus der Einzelzellaufschwemmung in einem Medium;
d) des In-Kontakt-Bringens zwischen dem mindestens einen Sphäroid und dem mindestens einen Therapeutikum;
e) des Messens des Ansprechens des mindestens einen Sphäroids auf das mindestens eine Therapeutikum.

12. Verfahren nach Anspruch 11, wobei der/die Biomarker aus der Gruppe der Protein-Biomarker, molekularen Biomarker und genomischen Biomarker ausgewählt ist/sind.

13. Verfahren nach Anspruch 11 oder 12, wobei das Therapeutikum gemäß dem erstellten Biomarkerprofil ausgewählt wird.

14. Verfahren nach einem der Ansprüche 11 bis 13, weiterhin umfassend den Schritt des Erstellens eines Biomarkerprofils des Sphäroids.

## Revendications

1. Procédé de test de la réponse de cellules à une exposition à au moins un produit thérapeutique, comprenant les étapes :
a) générer une suspension monocellulaire à partir d'un échantillon de tissu biologique obtenu directement sans les étapes intermédiaires de sous-culture et d'élimination des cellules apoptotiques de celle-ci ;
b) générer au moins un sphéroïde directement à partir de la suspension monocellulaire dans un milieu ;
c) mettre le ou les sphéroïdes en contact avec au moins un produit thérapeutique ;
d) mesurer la réponse du ou des sphéroïdes au ou aux produits thérapeutiques.

2. Procédé selon la revendication 1, dans lequel le tissu est issu d'un mammifère.

3. Procédé selon la revendication 1 ou 2, dans lequel l'échantillon de tissu biologique est choisi parmi un tissu sain, un tissu tumoral, un tissu d'un tissu bénin ou malin.

4. Procédé selon l'une quelconque des revendications 1 à 3, dans lequel le ou les composés biologiques et/ou chimiques sont choisis dans le groupe constitué des produits chimiothérapeutiques, des biomarqueurs et des produits thérapeutiques moléculaires.

5. Procédé selon l'une quelconque des revendications 1 à 4, dans lequel le ou les sphéroïdes sont également traités par radiothérapie et/ou hypothermie.

6. Procédé selon l'une quelconque des revendications 1 à 5, dans lequel la mise en contact du ou des sphéroïdes avec le ou les produits thérapeutiques dure de 12 à 120 heures.

7. Dispositif destiné à réaliser le procédé selon l'une quelconque des revendications 1 à 6, dans lequel le ou les sphéroïdes selon l'une quelconque des revendications 1 à 6 sont fermement fixés et/ou maintenus à l'intérieur d'un dispositif.

8. Dispositif selon la revendication 7, dans lequel le sphéroïde est un sphéroïde traité avec au moins un produit thérapeutique.

9. Dispositif selon la revendication 7 ou 8, **caractérisé en ce qu'**au moins un sphéroïde est fixé et/ou maintenu par inclusion dans un bloc de paraffine ou de glace carbonique.

10. Procédé de diagnostic d'individus qui répondent à une stratégie thérapeutique chimique ou biologique comprenant les étapes :
a) générer une suspension monocellulaire à partir d'un échantillon de tissu biologique obtenu directement sans les étapes intermédiaires de sous-culture et d'élimination des cellules apoptotiques de celle-ci ;
b) générer au moins un sphéroïde directement à partir de la suspension monocellulaire dans un milieu ;
c) mettre le ou les sphéroïdes en contact avec au moins un produit thérapeutique ;
d) mesurer la réponse du ou des sphéroïdes au ou aux produits thérapeutiques ;
dans lequel la réponse du ou des sphéroïdes au ou aux produits thérapeutiques est utilisée pour choisir un agent thérapeutique.

11. Procédé de définition d'un concept thérapeutique individuel pour un patient, comprenant les étapes :
a) exécuter un profil de biomarqueurs pour l'échantillon de tissu ;
b) générer une suspension monocellulaire à partir d'un échantillon de tissu biologique obtenu directement sans les étapes intermédiaires de sous-culture et d'élimination des cellules apoptotiques de celle-ci ;
c) générer au moins un sphéroïde directement à partir de la suspension monocellulaire dans un milieu ;
d) mettre le ou les sphéroïdes en contact avec au moins un produit thérapeutique ;
e) mesurer la réponse du ou des sphéroïdes au ou aux produits thérapeutiques.

12. Procédé selon la revendication 11, dans lequel le ou les biomarqueurs sont choisis dans le groupe comprenant les biomarqueurs protéiques, les biomarqueurs moléculaires et les biomarqueurs génomiques.

13. Procédé selon la revendication 11 ou 12, dans lequel le produit thérapeutique est choisi selon le profil de biomarqueurs exécuté.

14. Procédé selon l'une quelconque des revendications 11 à 13, comprenant en outre l'étape consistant à exécuter un profil de biomarqueurs du sphéroïde.
